Europäisches Patentamt

European Patent Office

Office européen des brevets

(19)

(11) Publication number: **0 164 007**
**B1**

(12) **EUROPEAN PATENT SPECIFICATION**

(45) Date of publication of patent specification: **09.11.88**

(21) Application number: **85106119.2**

(22) Date of filing: **18.05.85**

(51) Int. Cl.⁴: **G 02 B 27/04**

(54) **Portable viewing apparatus.**

(30) Priority: **08.06.84 US 618534**

(43) Date of publication of application:
**11.12.85 Bulletin 85/50**

(45) Publication of the grant of the patent:
**09.11.88 Bulletin 88/45**

(84) Designated Contracting States:
**DE FR GB IT**

(56) References cited:
**DE-A-2 431 461**
**DE-A-3 229 599**
**DE-B-1 797 305**
**DE-B-2 064 672**

(73) Proprietor: **GTI Graphic Technology, Inc.**
**P.O. Box 3138**
**Newburgh New York 12550 (US)**

(72) Inventor: **McCurdy, Frederic**
**38 Echo Lane**
**Newburgh New York 12550 (US)**

(74) Representative: **Zapfe, Hans, Dipl.-Ing.**
**Seestrasse 2 Postfach 30 04 08**
**D-6054 Rodgau-3 (DE)**

## Description

This invention relates to portable apparatus for simultaneously viewing a transparency and a reflection copy image. As used herein, the term "transparency" includes transparent or translucent positives or images through which light must pass. The term reflection copy image includes a front-viewed picture or other reflection subject matter such as, for example, a proof or print.

One prior apparatus for simultaneously viewing transparencies and reflection copy pictures is a stationary viewing station which accommodates a standard transparency viewer and into which a transparency viewer may be recessed. This viewing station has the limitation that it is not portable.

DE-A-2 431 461 and corresponding US-A-3 964 193 disclose a portable apparatus for simultaneously viewing transparencies and reflection copy images, which has the disadvantages that in one embodiment the portions of the apparatus must be inclined towards one another which makes viewing awkward, and that in the other embodiment a light source requires adjustment to a position for illuminating both the reflection copy image and the transparency. Both embodiments have the further disadvantage that stray-light is not sufficiently kept away from the images.

Another portable viewing apparatus for viewing only reflection copy images has the disadvantage that transparencies and reflection copy images cannot be simultaneously viewed and compared for color quality and uniformity.

It is an object of the present invention, therefore, to provide a new and improved portable apparatus for simultaneously viewing a transparency and a reflection copy image which avoids one or more of the disadvantages and limitations of prior viewing apparatus.

It is another object of the invention to provide a new and improved portable apparatus for simultaneously viewing a transparency and a reflection copy image which is housed in a protective case.

It is another object of the invention to provide a new and improved portable apparatus for simultaneously viewing a transparency and a reflection copy image in which the transparency is viewable at a convenient oblique angle with respect to the reflection copy image.

It is another object of the invention to provide a new and improved apparatus for simultaneously viewing a transparency and a reflection copy image which comply with American National Standard Institute standards, namely, ANSI PH2.31-1969 (R 1982) "Direct Viewing of Photographic Color Transparencies" and ANSI PH2.32-1972 (R 1981) "Viewing Conditions for the Appraisal of Color Quality and Color Uniformity in the Graphic Arts."

In accordance with the invention, a portable apparatus for simultaneously viewing a transparency and a reflection copy image comprises a portable case having a body portion having sides and adapted to stand approximately vertically and having a cover portion pivotally coupled to the body portion near the top of the body portion for approximately horizontal disposition upon opening the case. The apparatus includes a pair of side light-shield means individually pivotally coupled to the sides of the body portion for moving outwardly from the body portion to an approximately vertical disposition extending from the sides of the body portion. The apparatus also includes means pivotally coupled to the body portion approximately at the bottom thereof for moving outwardly from the body portion for approximately horizontal disposition. The body portion has a transparency viewing portion including back-lighting means for illuminating a transparency when disposed therein from behind. The cover portion includes lighting means for illuminating a reflection copy image when disposed on the approximately horizontally disposed means and for simultaneously illuminating the front of the transparency in the body portion.

For a better understanding of the present invention, together with other and further objects thereof, reference is made to the following description, taken in connection with the accompanying drawings, and its scope will be pointed out in the appended claims.

Referring now to the drawings:

Fig. 1 is a perspective view of portable apparatus for simultaneously viewing a transparency and a reflection copy image, constructed in accordance with the invention;

Fig. 2 is a side elevational view of the Fig. 1 apparatus;

Fig. 3 is a perspective view of the Fig. 1 apparatus in a fully open position;

Fig. 4 is a front elevational view of the Fig. 1 apparatus in an operable open position; and

Fig. 5 is a sectional view of the Fig. 1 apparatus, taken along line 5-5 of Fig. 4.

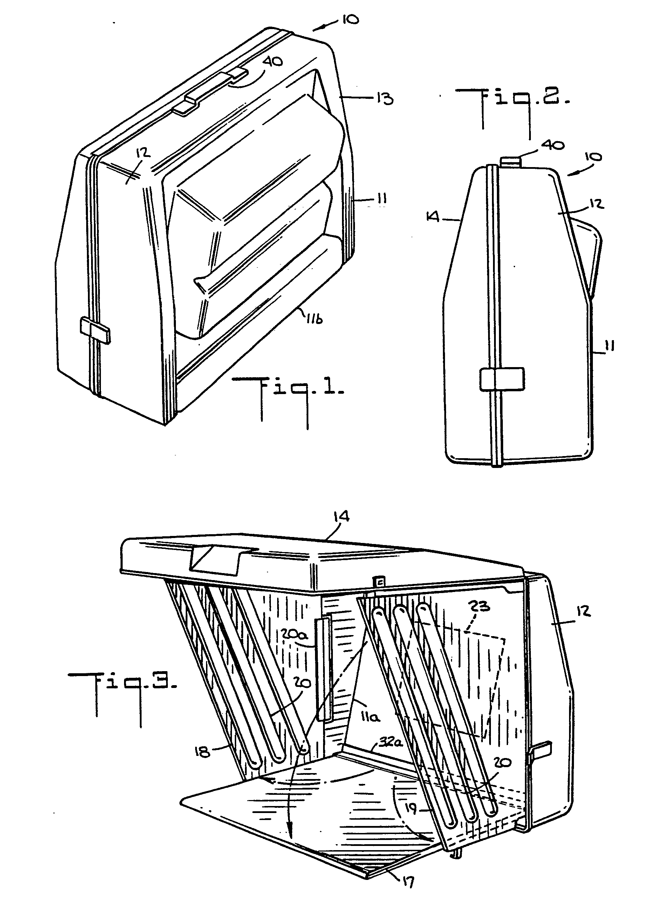

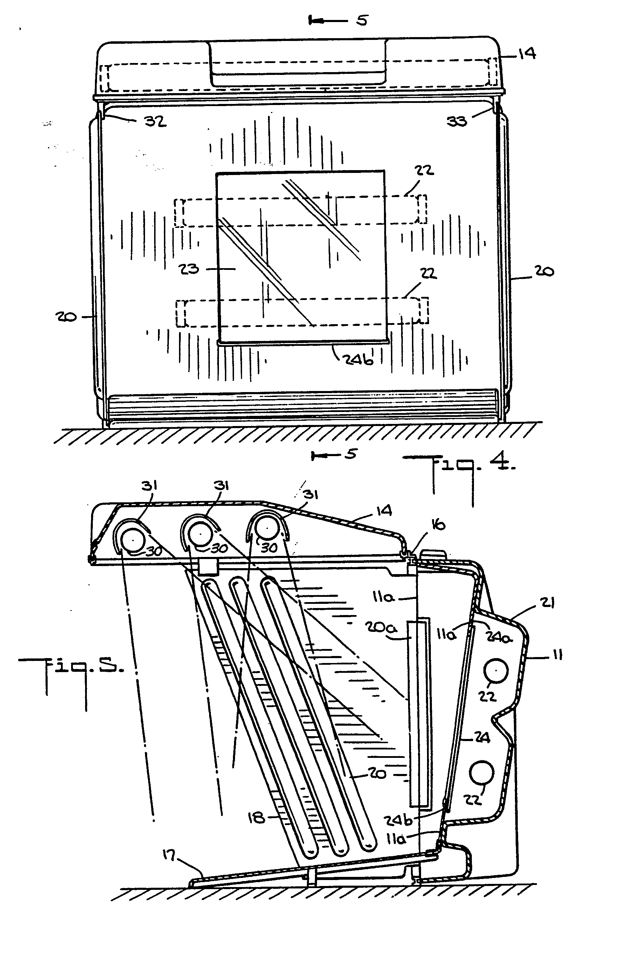

Referring now more particularly to Figs. 1 and 2 of the drawings, a portable apparatus for simultaneously viewing a transparency and a reflection copy image, constructed in accordance with the invention, comprises a portable case 10 having a body portion 11, preferably of a suitable plastic material, having sides 12, 13 and adapted to stand approximately vertically. The portable case 10 has a cover portion 14, also preferably of the same plastic material, pivotally coupled to the body portion near the top of the body portion for approximately horizontal disposition upon opening the case as represented in Figs. 3, 4 and 5.

The cover portion 14 and the body portion 11 include hinged interconnections 16 (Fig. 5) for moving the cover portion to the approximately horizontal disposition.

The apparatus also includes a pair of side light-shield means 18, 19 individually pivotally coupled to the sides 12, 13 of the body portion 11 for moving outwardly from the body portion 11 to an approximately vertical disposition extending from the sides of the internal member 11a of the body portion 11. The pair of light-shield means 18, 19 preferably comprise opaque plates having reinforcing ribs 20 and connected by any suitable hinges, for example, hinge 20a, to the sides of the internal member lla. As represented in Fig. 4, the plates 18, 19 are effective to support the cover portion 14 in its approximately horizontal disposition by means of connectors 32, 33 in the cover portion 14 which rest on the tops of the plates 18, 19.

The apparatus also includes means 17 pivotally coupled to an internal member 11a, which is attached to and included in the body portion 11, approximately at the bottom of body portion 11, for moving outwardly from the body portion 11 for approximately horizontal disposition onto a pivotal member 32a, as represented in Fig. 3. The first means 17 preferably comprises a plate on which a reflection image may be placed between the side plates 18, 19. The term "approximately horizontal" disposition when referring to the plate 17 includes small angular dispositions of less than 25 degrees, for example, 7 degrees, as represented in Fig. 5, which reduces glare.

Referring now to Figs. 4 and 5, the body portion 11 has a transparency viewing portion 21 including back-lighting means 22 for illuminating from behind a transparency 23 when disposed therein. The transparency viewing portion preferably includes a surface 24 at an oblique angle to the approximately vertically disposed body portion 11 for determining the angle at which the transparency is disposed for viewing. The surface 24 preferably is a translucent acrylic surface supported by an opaque plate portion 24a of the internal member 11a by any suitable means, for example, an adhesive. The plate portion 24a preferably has a trough 24b in which the bottom of the transparency may be placed for support. The back-lighting means 22 preferably comprises two fluorescent lights of predetermined intensity and color quality, such that the resultant illumination on the transparency viewer surface is D5000. (American National Standard Institute standard).

The cover portion includes lighting means for illuminating a reflection copy image when disposed on the approximately horizontally disposed means 17 and for simultaneously illuminating the front of the transparency 23 in the body portion. The lighting means preferably comprises three fluorescent bulbs 30 of color quality (D5000) and reflector means for reflecting light onto the reflection copy image and onto the front of the transparency. The reflector means may, for example, comprise shaped, adjustable metal reflectors 31, two of which are directed toward the transparency and the reflection copy image, and one of which is directed toward the reflection copy image.

A suitable ballast (not shown) for the lighting means 22 and 30 may be included in the body portion 11 which may include a suitable ventilating portion 11b (Fig. 1).

As will be apparent from the foregoing description, the portable apparatus may be carried by a suitable handle 40 and the body portion 11 may be placed in the vertical position represented in the drawings. The cover 14 may then be opened to an approximately horizontal position. The opaque plates 18, 19 may then be opened to the position represented in Fig. 3 in broken line construction and in Figs. 4 and 5. The plate 17 may then be folded to its approximately horizontal position, as represented in Fig 3. A transparency may be placed on the surface of the transparency viewer and a reflection copy image may be placed on the plate 17. The fluorescent lights are illuminated to provide back lighting for the transparency and the fluorescent lights and reflectors provide the same color quality (D5000) on the transparency and the reflection copy image and with such intensities thereon as to comply with the above-mentioned American National Standard Institute standards. The plates 18, 19 eliminate unwanted light and colors from striking the reflection copy image or the transparency from the sides of the apparatus. This combination renders comparison of the color quality practical in accordance with the above-mentioned American National Standard Institute standards.

The apparatus may be closed by reversing the steps described above for opening the same.

## Claims

1. A portable apparatus for simultaneously viewing a transparency and a reflection copy image comprising:

a portable case (10) having a body portion (11) having sides (12, 13) and adapted to stand approximately vertically and having a cover portion (14) pivotally. coupled to said body portion (11) near the top of said body portion (11) for approximately horizontal disposition upon opening the portable case (10);

a pair of side light-shield means (18, 19) individually pivotally coupled to said sides (12, 13) of said body portion (11) for moving outwardly from said body portion (11) to an approximately vertical disposition extending from said sides (12, 13) of said body portion (11).

support means (17) pivotally coupled to said body portion (11) near the bottom thereof for moving outwardly from said body portion (11) for approximately horizontal disposition;

said body portion (11) having a transparency viewing portion (21) including back-lighting means (22) for illuminating a transparency when disposed therein from behind and

said cover portion (14) including lighting means (30) for illuminating a reflection copy image when disposed on said approximately horizontally disposed support means (17) and for simultaneously illuminating the front of the transparency in said body portion (11).

2. A portable apparatus in accordance with claim 1 in which said cover portion (14) and said body portion (11) include hinged interconnections (16) for moving said cover portion (14) to said approximately horizontal disposition.

3. A portable apparatus in accordance with claim 1 in which said pair of light-shield means (18, 19) individually comprise opaque plates.

4. A portable apparatus in accordance with claim 3 in which said light-shield means (18, 19) are effective to support said cover portion (14) in said approximately horizontal disposition.

5. A portable apparatus in accordance with claim 1 in which said support means (17) pivotally coupled to said body portion (11) approximately at the bottom thereof comprises a plate.

6. A portable apparatus in accordance with claim 1 in which said transparency-viewing portion (21) includes a surface (24) at an oblique angle to said approximately vertically disposed body portion (11) for determining the angle at which the transparency is disposed for viewing.

7. A portable apparatus in accordance with claim 6 which includes a plate portion (24a) having a trough (24b) therein for supporting a transparency when disposed therein against said oblique surface (24).

8. A portable apparatus in accordance with claim 1 in which said lighting means (30) included in said cover portion (14) comprises reflector means (31) for directing light onto the reflection copy image and onto the front of the transparency.

**Patentansprüche.**

1. Tragbarer Betrachtungsapparat für das gleichzeitige Betrachten eines Transparent-Bildes und einer reflektierenden Reproduktion gekennzeichnet durch:

einen Tragkoffer (10) mit einem Hauptteil (11), Seitenwänden (12, 13), mit Mitteln zur angenähert senkrechten Aufstellung und mit einem Deckelteil (14), das in der Weise in der Nähe der Oberseite des Hauptteils (11) mit diesem verbunden ist, daß es bei geöffnetem Tragkoffer (10) angenähert waagrecht ausgerichtet ist;

ein Paar seitlicher Lichtabschirmungen (18, 19), die zum Zwecke einer Auswärtsbewegung gegenüber dem Hauptteil (11) in eine angenähert senkrechte, sich von den Seitenwänden (12, 13) des Hauptteils (11) weg erstreckende Lage einzeln schwenkbar mit den Seitenwänden (12, 13) des Hauptteils (11) verbunden sind;

ein Auflageteil (17), das in der Nähe des Bodens des Hauptteils (11) zum Zwecke einer Auswärtsbewegung gegenüber dem Hauptteil (11) in eine angenähert waagrechte Lage schwenkbar mit dem Hauptteil (11) verbunden ist;

ein zum Hauptteil (11) gehörendes Transparent-Betrachtungsteil (21) einschließlich einer Rückseiten-Beleuchtung (22) für die Beleuchtung eines eingelegten Transparents von der Rückseite her und

eine zum Deckelteil (14) gehörende Beleuchtungseinrichtung (30) für die Beleuchtung einer auf dem angenähert waagrecht ausgerichteten Auflageteil (17) befindlichen reflektierenden Reproduktion und für die gleichzeitige Beleuchtung der Vorderseits des im Hauptteil befindlichen Transparent-Bildes.

2. Tragbarer Betrachtungsapparat nach Anspruch 1, bei dem das Deckelteil (14) und das Hauptteil (11) Scharniere (16) für die Bewegung des Deckelteile (14) in die besagte angenähert waagrechte Stellung zwischen sich einschließen.

3. Tragbarer Betrachtungsapparat nach Anspruch 1, bei dem das besagte Paar von Lichtabschirmungen (18, 19) jeweils aus undurchsichtigen Platten besteht.

4. Tragbarer Betrachtungsapparat nach Anspruch 3, bei dem die besagten Lichtabschirmungen (18, 19) zur Abstützung des Deckelteils (14) in der angenähert waagrechten Lage ausgebildet sind.

5. Tragbarer Betrachtungsapparat nach Anspruch 1, bei dem das besagte Auflageteil (17), das mit dem Hauptteil (11) in dessen Bodenbereich schwenkbar verbunden ist, eine Platte bildet.

6. Tragbarer Betrachtungsapparat nach Anspruch 1, bei dem das besagte Transparent-Betrachtungsteil (21) eine Auflagefläche (24) besitzt, die zum Zwecke einer Festlegung des Betrachtungswinkels für das Transparent-Bild unter einem spitzen Winkel zum besagten, angenähert senkrecht angeordneten Hauptteil (11) verläuft.

7. Tragbarar Betrachtungsapparat nach Anspruch 6, bei dem ein Plattenteil (24a) mit einer Rinne (24b) für die Unterstützung eines gegen die schiefgestellte Auflagefläche (24) gelegten Transparatent-Bildes vorgesehen ist.

8. Tragbarar Betrachtungsapparat nach Anspruch 1, bei dem die in dem besagten Deckelteil (14) angeordnete Beleuchtungseinrichtung (30) Reflektoren (31) für die Leitung des Lichts auf die reflektierende Reproduktion und auf die Vorderseite des Transparent-Bildes aufweist.

**Revendications**

1. Dispositif portable pour l'observation simultanée d'un cliché transparent et d'une image de reproduction réfléchissante, comprenant:

un boîtier portable (10) comportant un corps (11) qui possède des côtés (12, 13) et qui peut se tenir sensiblement verticalement, un couvercle (14) étant relié de façon pivotante audit corps (11) près du sommet dudit corps (11) de manière à prendre une position sensiblement horizontale lors de l'ouverture du boîtier portable (10);

deux éléments latéraux (18, 19) faisant écran à la lumière, reliés individuellement de façon pivotante aux dits côtés (12, 13) dudit corps (11) de manière à se déplacer vers l'extérieur par rapport audit corps (11) jusqu'à une position sensiblement verticale partant desdits côtés (12, 13) dudit corps (11);

un élément support (17), relié de façon pivotante audit corps (11) près de la base de celui-ci de manière à se déplacer vers l'extérieur par rapport audit corps (11) et à prendre une position sensiblement horizontale;

ledit corps (11) comprenant une partie (21) d'observation de cliché transparent qui comporte des moyens d'éclairage arrière (22) pour éclairer un cliché transparent disposé dans cette partie, par l'arrière; et

ledit couvercle (14) comprenant des moyens d'éclairage (30) pour éclairer une image de reproduction réfléchissante placée sur ledit élément support disposé sensiblement horizontalement (17), et pour éclairer simultanément l'avant du cliché transparent placé dans ledit corps (11).

2. Dispositif portable suivant la revendication 1, dans lequel ledit couvercle (14) et ledit corps (11) comportent des charnières d'interconnexion (16) permettant d'amener ledit couvercle (14) à ladite position sensiblement horizontale.

3. Dispositif portable suivant la revendication 1, dans lequel les deux éléments (18, 19) formant écran à la lumière comprennent individuellement des plaque opaques.

4. Dispositif portable suivant la revendication 3, dans lequel lesdits éléments formant écran à la lumière (18, 19) servent à supporter ledit couvercle (14) dans ladite position sensiblement horizontale.

5. Dispositif portable suivant la revendication 1, dans lequel ledit élément support (17), relié de façon pivotante audit corps (11) sensiblement à la base de ce dernier, comprend une plaque.

6. Dispositif portable suivant la revendication 1, dans lequel ladite partie (21) d'observation de cliché transparent comprend une surface (24) inclinée par rapport audit corps (11) placé sensiblement verticalement, pour déterminer l'angle suivant lequel le cliché transparent est disposé pour l'observation.

7. Dispositif portable suivant la revendication 6, qui comprend une partie de plaque (24a) comportant un canal (24b) pour supporter un cliché transparent disposé dans ce canal et contre ladite surface oblique (24).

8. Dispositif portable suivant la revendication 1, dans lequel les moyens d'éclairage (30) inclus dans ledit couvercle (14) comprennent des moyens de réflexion (31) pour diriger la lumière sur l'image de reproduction réfléchissante et sur l'avant du cliché transparent.

0 164 007

Fig.1.

Fig.2.

Fig.3.

1

0 164 007

Fig. 4.

Fig. 5.

2